# EUROPÄISCHE PATENTANMELDUNG

(11) **EP 2 583 957 A1**
(43) Veröffentlichungstag der Anmeldung: **24.04.2013**
(21) Anmeldenummer: 11185644.9
(22) Anmeldetag: 18.10.2011
(51) Int. Cl.: C07C 1/24, C07C 11/09, C07C 11/08

(54) **Lineare Butene aus Isobutanol**

(71) Anmelder: LANXESS Deutschland GmbH, 51369 Leverkusen (DE)
(72) Erfinder: Boll, Matthias, 51061 Köln (DE); Föllinger, Thomas, 41539 Dormagen (DE)

(57) **Zusammenfassung**

Die vorliegende Erfindung betrifft die Herstellung linearer Butene aus Isobutanol, das bevorzugt mikrobiell und/oder fermentativ aus nachwachsenden Rohstoffen gewonnen wurde.

## Beschreibung

Die vorliegende Erfindung betrifft die Herstellung linearer Butene aus Isobutanol, das bevorzugt mikrobiell und/oder fermentativ aus nachwachsenden Rohstoffen gewonnen wurde.

Lineare Butene im Sinne der vorliegenden Erfindung sind bevorzugt Buten-1 und Buten-2 (von letzterem sowohl die trans- wie auch die cis-Form), insbesondere bevorzugt Buten-1.

Isobutanol gehört zu den chemischen Verbindungen, die sich durch gezielte biochemische Prozesse aus Naturprodukten herstellen lassen und die aufgrund ihres C4-Grundkörpers das Interesse der chemischen Industrie auf sich ziehen. Aus Isobutanol lässt sich durch Dehydratisierung Isobuten herstellen, das einen wichtigen Baustein für die Synthese von Lösungsmitteln und für Kraftstoffadditive wie zum Beispiel Methyl-t-butylether (MTBE) darstellt und bei dem es sich außerdem um einen wichtigen Rohstoff in der Kunststoffindustrie handelt. Sehr großes Interesse herrscht zusätzlich an linearen Butenen, da sich diese sowohl direkt in den Polymerisationsprozessen der Kunststoffindustrie einsetzen lassen, als auch, zum Beispiel durch eine partielle Oxidation, in den wichtigen Rohstoff Butadien überführen lassen.

Aus GB 576 480 ist die Synthese von Butylen aus sek.-Butylalkohol bei 230°C in Gegenwart von Salzsäure oder Eisenchlorid bekannt.

Auch die Dehydratisierung von Isobutanol zu einem Isobuten / n-Buten Gemisch durch Säuren (Schwefelsäure-/Phosphorsäuregemisch) in flüssiger Phase ist schon lange bekannt, wobei der Anteil der linearen Butene sehr unterschiedlich war und beispielsweise bis zu 66 % betrug (Canadian Journal of Chemistry (1970), 48(22), 3545-8). Die gesamte Ausbeute gasförmiger Produkte mit 15-45 % in diesen Beispielen war allerdings für eine wirtschaftliche Umsetzung viel zu gering.

Um die Reaktion technisch besser umsetzen zu können, wurden Versuche unternommen, Schwefelsäure auf Träger wie zum Beispiel γ-Aluminiumoxid aufzuziehen und mit diesen die Umsetzung in einem Festbettreaktor durchzuführen. So wird beispielsweise in Applied Catalysis, A.: General (2001, 214(2), 251-257) beschrieben, dass sich bei der Dehydratisierung von Isobutanol das Isomerenverhältnis zu Gunsten der linearen Butene verschiebt, wenn die Temperatur auf über 400°C angehoben wird. Dort werden Selektivitäten von 53,4 % n-Butene zu 40 % iso-Buten (bei 400°C) und 44,7 % n-Butene zu 39,8 % iso-Buten (470°C) gefunden. Zum einen wurde jedoch bei sehr hohen Temperaturen gearbeitet, die nur wenig wirtschaftlich sind. Zum anderen wurde das Isobutanol, bevor es in den eigentlichen Reaktor dosiert wurde, durch Glaswolle geleitet, die mit rund 300°C bereits sehr heiß war. Nach den im Rahmen der vorliegenden Erfindung gefundenen Erfahrungen hat aber SiO₂ selber bereits einen großen Einfluss auf die Selektivität der Dehydratisierung.

Die Reaktion von Isobutanol zu Isobuten an festen Katalysatoren ohne weitere Zusätze wurde bereits mehrfach beschrieben. So wird zum Beispiel in DD 245866 generell der Einsatz von Aluminiumoxid, SiO₂, CaHPO₄ und AIPO₄ erwähnt. Allerdings wird nicht auf einen möglichen Einfluss dieser Katalysatoren auf das Isomerenverhältnis eingegangen. Eine besonders gute Selektivität in Richtung Isobuten wird mit reinem γ-Aluminiumoxid erreicht (US 20100216958A). Technische Ausführungen hierzu werden ebenfalls beschrieben (Proceedings - Annual International Pittsburgh Coal Conference (1993), 10th, 1196-9).

All diese Verfahren verwenden Isobutanol als Edukt, das nicht durch bevorzugt mikrobielle oder fermentative Prozesse gewonnen wurde oder bedienen sich zumeist der flüssigen Katalyse.

Schließlich wurde in WO 2008/016428 A2 und WO 2008/066581 A1 der Einfluss verschiedenster saurer Katalysatoren auf die Reaktion von 2-Butanol zu Butenen untersucht. Dabei kamen als Katalysator Schwefelsäure, Wolframsäure, sulfatiertes Zirkonium, Amberlyst^{®}15, 13 % Nafion^{®}/SiO₂, CBV3020 E oder H-Mordenite zum Einsatz. 2-Butanol ist aber nicht der bevorzugte Einsatzstoff, da aus ihm nicht auch Isobuten gewonnen werden kann.

Aus Top. Catal. (2010) 53:1224 - 1230 ist die Dehydratisierung von fermentativ gewonnenem Isobutanol mittels eines Katalysators BASF AL3996, einem Aluminiumoxid mit einer BET-Oberfläche von 200 m²/g, bekannt. Das Hauptprodukt ist hier jedoch Isobuten als Rohstoff für synthetische Kraftstoffe. Lineare Butene fallen dabei insbesondere bei niedrigen Temperaturen nur in geringem Maße an.

Aufgabe der vorliegenden Erfindung war es daher, ausgehend von bevorzugt fermentativ oder mikrobiell gewonnenem Isobutanol einen Katalysator zu entwickeln, der lineare Butene, insbesondere Buten-1, über einen langen Zeitraum in wirtschaftlichen Mengen liefert.

Lösung der Aufgabe und Gegenstand der vorliegenden Erfindung ist ein Verfahren zur Dehydratisierung von Isobutanol zu linearen n-Butenen, dadurch gekennzeichnet, dass als Katalysator in einem Festbett TiO₂ mit einer hohen BET-Oberfläche > 10m²/g oder Al₂O₃/SiO₂ eingesetzt werden. Bevorzugt beträgt die BET-Oberfläche des TiO₂-Katalysators 20 bis 400 m²/g, besonders bevorzugt 40 bis 200 m²/g, ganz besonders bevorzugt 140 bis 160 m²/g. Bevorzugt beträgt die BET-Oberfläche des in einer alternativen Ausführungsform einzusetzenden Katalysators Al₂O₃/SiO₂ 0,1 bis 100m²/g. Die BET-Oberflächen-Werte im Rahmen der vorliegenden Erfindung sind zu bestimmen durch Adsorption in Stickstoff nach DIN 66131.

Zur Klarstellung sei angemerkt, dass vom Rahmen der Erfindung alle aufgeführten, allgemeinen oder in Vorzugsbereichen genannten Definitionen und Parameter in beliebigen Kombinationen umfasst sind.

Als Edukt wird Isobutanol, bevorzugt aus mikrobiellen und/oder fermentativen Prozessen, wie sie beispielsweise in WO 2009/086423A2; WO 2010/151832A1, WO 2010/151525A1, US 2010/0216958A1, US 2007/0092957A1 oder DE 10 2008 010 121 A1 beschrieben werden, eingesetzt. Ein aus fermentativem oder mikrobiellem Herstellprozess gewonnenes Isobutanol das für den erfindungsgemäßen Einsatz verwendet werden kann, ist gemäß US 2010/0216958A1 Beispiel 1 bevorzugt ein Stoffgemisch enthaltend 98 - 99,9 Gew.-% Isobutanol und 2 - 0,1 Gew.-% Wasser, bevorzugt 98,5 - 99,9 Gew.-% Isobutanol und 1,5 - 0,1 Gew.-% Wasser. In einer besonders bevorzugten Ausführungsform kann im erfindungsgemäß einzusetzenden Stoffgemisch neben Isobutanol und Wasser noch 3-Methyl-1-butanol zu 0,5 bis 1,5 Gew.-% enthalten sein. Daraus ergeben sich Stoffgemische enthaltend 98 - 99,8 Gew.-% Isobutanol, 0,1 - 1,9 Gew.-% 3-Methyl-1-butanol und 0,1 - 1,9 Gew.-% Wasser, bevorzugt 98,5 - 99,8 Gew.-% Isobutanol, 0,1 - 1,75 Gew.-% 3-Methyl-1-butanol und 0,1 - 1,75 Gew.-% Wasser.

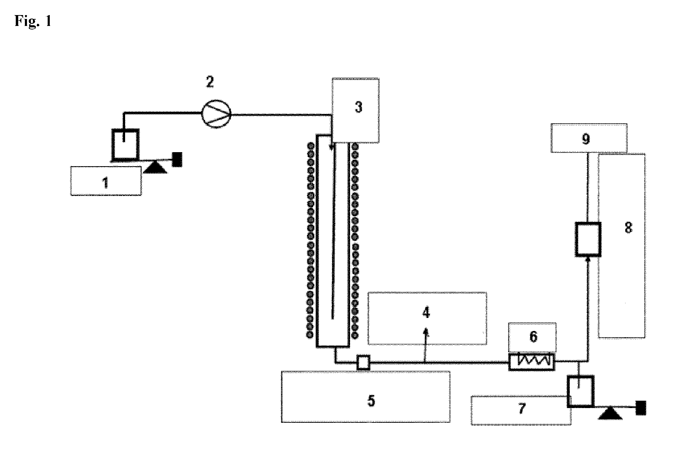

Das erfindungsgemäß einzusetzende Festbett ist bevorzugt Teil eines Rohrbündelreaktors oder Röhrenreaktors, der wiederum bevorzugt Bestandteil einer Apparatur, dargestellt in Fig. 1, ist. Der erfindungsgemäß einzusetzende Festbettrohrreaktor ist bevorzugt ein Rohrbündelreaktor oder Röhrenreaktor aus Stahl oder Edelstahl. Bei diesen Reaktoren befindet sich der Katalysator als Festbett in einem Reaktionsrohr oder in einem Bündel von Reaktionsrohren. Die Reaktionsrohre werden üblicherweise dadurch indirekt beheizt, indem der die Reaktionsrohre umgebende Raum durch Verbrennen eines Gases, beispielsweise Methan, beheizt wird. Günstig ist es dabei, diese indirekte Form der Aufheizung lediglich auf die ersten ca 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Weitere Arten der Beheizung sind dem Fachmann bekannt. Übliche Reaktionsrohr-Innendurchmesser betragen 2 bis 15cm. Ein typischer Rohrbündelreaktor umfasst ca. 300 bis 1000 Reaktionsrohre. Derartige Reaktoren sind Stand der Technik und werden beispielsweise bei der MAN DWE GmbH in Deggendorf, Deutschland gefertigt. Diese weisen einen (Edel-) stahllochboden. auf, worauf die Schüttung mit Katalysator gegeben wird. Erfindungsgemäß wird der Reaktor bevorzugt bei einem Druck von 1 bis 300 bar, besonders bevorzugt bei 1 bis 100 bar, ganz besonders bevorzugt bei 1 bis 10 bar, insbesondere bevorzugt bei 1 bis 6 bar und insbesondere ganz besonders bevorzugt bei 3 bis 6 bar betrieben.

Die Dehydratisierung des Isobutanols in der Gasphase im Festbett wird bei Bedingungen durchgeführt, die für diese Reaktion im Allgemeinen bekannt sind. Dazu verwendet man bevorzugt Temperaturen im Bereich von etwa 200 bis 500°C, vorzugsweise 250 bis 400°C. Erfindungsgemäß wird TiO₂ mit oben beschriebener BET-Oberfläache als Katalysator im Festbett eingesetzt. Erfindungsgemäß wird in einer bevorzugten Ausführungsform Al₂O₃/SiO₂ mit oben beschriebener BET-Oberfläche und mit einem Gehalt an SiO₂ im Bereich von 0,1 bis 80 Gew.-% als Katalysator im Festbett eingesetzt. Insbesondere bevorzugt wird TiO₂ mit der oben beschriebenen BET-Oberfläche eingesetzt. Insbesondere ganz besonders bevorzugt wird als Katalysator TiO₂ mit den oben angegebenen BET-Oberflächen-Werten vom Typ Anatas eingesetzt. Die Katalysatorgeometrie ist bevorzugt kugelförmig oder zylindrisch, hohl oder voll.

Das erfindungsgemäße Verfahren kann kontinuierlich oder diskontinuierlich betrieben werden. Bevorzugt wird es kontinuierlich betrieben.

Technisch kann das Produktgemisch in seine einzelnen Komponenten aufgetrennt werden, wie es zum Beispiel in DE 10 2005 062 700 A1 beschrieben wird. Dabei wird beispielsweise das Nebenprodukt Isobuten mittels saurer Katalysatoren mit einem Alkohol zu tert.-Butylethern umgesetzt und von den linearen Butenen abgetrennt bevor Letztere schließlich destillativ aufgearbeitet werden.

### Experimenteller Teil

Die Arbeiten im Rahmen der vorliegenden Erfindung wurden in einer Laborapparatur gemäß Fig. 1 und mit einem Labor-Rohrreaktor mit einem Edelstahlrohr einer Länge von ca. 1000 mm und einem Rohrquerschnit von 20mm durchgeführt. Das Edelstahlrohr wies einen Edelstahllochboden auf. In das Stahlrohr wurden zunächst Edelstahlkugeln, bevorzugt mit 2-3 mm Durchmesser, als Platzhalter eingefüllt. Auf diese Schüttung wurde der Katalysator gefüllt, der aus Formkörpern, bevorzugt aus Extrudaten oder Kugeln in von etwa 3mm Länge bzw. Durchmesser, bestand, um den Druckverlust über das Rohr zu verringern. Auf das Katalysatorbett wurden schließlich kleine Rollen von Drahtgewebe, bevorzugt mit einem Durchmesser und einer Länge der Rollen von ca. 5 mm, bis zur Oberkante des Reaktorrohres 3 in Fig.1 eingefüllt. Der Festbettreaktor wurde in einen Ofen eingebaut, der das Reaktorrohr auf der Länge von Fritte (Edelstahllochboden) bis Reaktorrohroberkante elektrisch beheizt. Durch Einbau von fünf Thermoelementen in die Achse des Rohres konnte die Innentemperatur oberhalb und unterhalb der Katalysatorschüttung sowie am Anfang, in der Mitte und am Ende des Bettes gemessen werden. Das Reaktorrohr wurde bei einem Druck von 1 bis 6,5 bar(a), bevorzugt bei 3 bis 5 bar(a), betrieben. Sichergestellt werden konnte dies durch Einbau eines elektrisch über den Innendruck geregelten Druckhalteventils 5 in Fig.1 am heißen Reaktorausgang. Danach wurde das noch heiße Reaktionsgas entnommen und direkt in einem Gaschromatographen 4 in Fig.1 analysiert. Die Probennahme erfolgte über beheizte Kapillarleitungen. Die Trennung der Komponenten erfolgte gaschromatographisch und mittels Flammenionisation. Die Apparatur gemäß Fig.1 enthielt ferner ein Isobutanol-Vorlagegefäss 1 auf einer Waage, eine Pumpe 2 in Fig.1 zur Dosierung von Isobutanol in den Festbettreaktor 3 in Fig.1, einen Kühler 6 in Fig.1 zur Wasserkondensation, eine Waage 7 in Fig.1 zum Auswiegen des Reaktionswassers, einen Probenzylinder 8 in Fig.1 zur Probenentnahme für die Offline-Gasanalytik und den Auslaß 9 in Fig.1 aus der Apparatur zum Abzug. Bar(a) oder bara bedeutet im Sinne der vorliegenden Erfindung der "absolute Druck" der sich aus 1 bar Atmosphärendruck plus entsprechendem Überdruck ergibt. Siehe hierzu auch http://de.wikipedia.org/wikiBar_(Einheit).

Als Edukt wurde Isobutanol, hergestellt auf Basis von Rohöl verwendet, das >95 Gew.-% Isobutanol enthielt, eingesetzt.

### Beispiele

Die folgenden Beispiele sollen die Erfindung exemplarisch beschreiben, ohne sie einzuschränken.

### Vergleichsbeispiel

Zum Vergleich wird ein ähnliches Verfahren vorgestellt, wobei als Katalysator Aluminiumoxid eingesetzt wurde.

### Allgemeiner Aufbau:

In ein Rohr aus Edelstahl mit einer Länge von ca. 1000 mm und mit einem Edelstahllochboden wurden ca. 210 mm Stahlkugeln (ca. 2-3 mm Durchmesser) eingefüllt. Auf diese Schüttung wurde der Katalysator gefüllt, der aus Formkörpern (zum Beispiel Extrudate mit ca. 3mm Länge und Dicke oder Kugeln von einem Duchmesser von ca. 3 mm) bestand, um den Druckverlust über das Rohr zu verringern. Auf das Katalysatorbett wurden nun kleine Rollen von feinem Drahtgewebe (Durchmesser und Länge der Rollen liegt bei ca. 5 mm) bis zur Oberkante des Reaktorrohres eingefüllt. Der Reaktor wurde in einen Ofen eingebaut, der das Reaktorrohr auf der Länge von Fritte bis Reaktorrohroberkante elektrisch beheizte. Durch Einbau von fünf Thermoelementen in die Achse des Rohres konnte die Innentemperatur oberhalb und unterhalb der Katalysatorschüttung sowie am Anfang, in der Mitte und am Ende des Bettes gemessen werden. Das Reaktorrohr wurde zum Beispiel bei Normaldruck, unter einem Druck von 5 bara oder unter Druck bei 6,5 bara betrieben. Sichergestellt wurde dies durch Einbau eines elektrisch über den Innendruck geregelten Ventils am heißen Reaktorausgang. Es wurde das noch heiße Reaktionsgas aus der drucklosen Gasphase direkt hinter diesem Ventil entnommen und direkt in einem Gaschromatographen analysiert. Für diese Analysen wurde ein Agilent GC 7890 mit GICU-Ventil (Gasinjektionsventil) der Firma JAS mit einer beheizten 250µl Probenschleife zur Analytik verwendet. Die Probennahme erfolgte über beheizte Kapillarleitungen aus Edelstahl. Die Trennung der Komponenten erfolgte auf einer Varian CP-Wax 52CB (25m * 0,25mm * 0,2µm), wobei der unpolare Leichtsiederanteil mittels eines Dean-Switches auf eine Agilent HP-AL/KCL (30m * 0,250mm * 5,00µm) zur Isomerentrennung umgeleitet wurde und mittels Flammenionisation detektiert wurde. Die übrigen Komponenten auf der CP-Wax wurden parallel am Flammenionisationsdetektor und am Wärmeleitfähigkeitsdetektor (für das Wasser) detektiert.

Eine für das Verfahren erforderliche Apparatur ist in Fig. 1 dargestellt. In Fig. 1 bedeuten:
1 = Isobutanol-Vorlagegefäß auf einer Waage
2 = Pumpe zur Dosierung von Isobutanol in Reaktor 3
3 = Festbettreaktor aus Edelstahl (1.4571) mit einem Innendurchmesser von ca. 18 mm
4 = Online Gaschromatograph
5 = Druckhalteventil
6 = Kühler zur Wasserkondensation
7 = Reaktionswasser (wird ausgewogen)
8 = Probenzylinder zur Probenentnahme für die Offline-Gasanalytik
9 = Auslaß zum Abzug

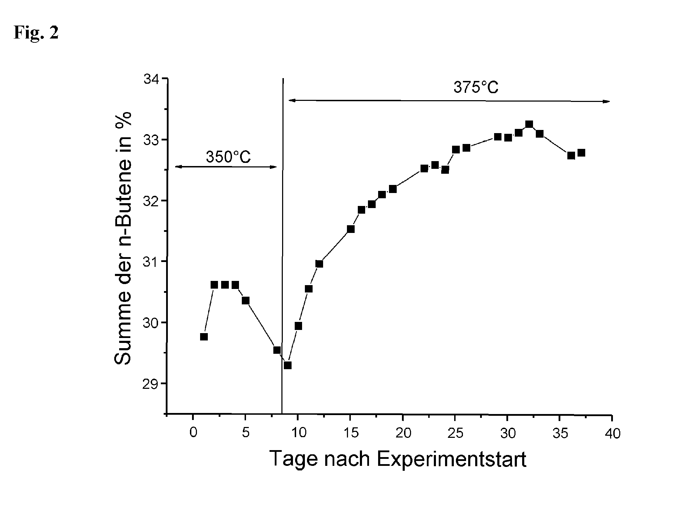

Fig. 2 zeigt den Anteil der Summe der n-Butene (GC-Flächenprozente, ermittelt wie im experimentellen Teil beschrieben) im Reaktionsprodukt ohne Berücksichtigung der Wasseranteile des gemäß des erfindungsgemäßen Verfahrens entstandenen n-Butens aus Isobutanol im Langzeittest mit einem TiO₂-Katalysator von St.-Gobain NorPro (Typ XT25376). Die Versuchsdurchführung entsprach dabei im Aufbau dem Beispiel 1, der Druck wurde allerdings im Reaktorrohr unverändert bei 6,5 bar absolut belassen und nur die Temperatur einmal variiert. Fig. 2 stellt damit die Ergebnisse aus Beispiel 3 graphisch dar.

### Erfindungsgemäß eingesetzt wurden folgende Katalysatoren:

XT25376 von St-Gobain-NorPro, TiO₂ mit einer BET-Oberfläche von ca. 140 m²/g und im Anatas-Typ

Al₂O₃/SiO₂ -Mischoxid, das zu 24,3 Gew.-% aus Al₂O₃ und zu 74,2 Gew.-% aus SiO₂ besteht und eine BET-Oberfläche von 434 m²/g besaß (Hersteller: St-Gobain NorPro, Versuchnummer 2006120085 DMS 2010 - 0380)

ST61120 von St-Gobain NorPro, TiO₂ mit einer BET-Oberfläche von 156 m²/g (CAS 13463-67-7)

Es zeigt sich, dass der Gehalt an n-Butenen mit steigendem Druck zunimmt, so dass nach dem erfindungsgemäßen Verfahren - je nach Bedarf - Isobuten und/oder n-Buten hergestellt werden kann. Technisch können die Komponenten dann voneinander getrennt werden , wie zum Beispiel in DE 10 2005 062 700 A1 beschrieben.

### Beispiel 1:

In einem Aufbau wie in Fig. 1 beschrieben wurde das Reaktorrohr in folgender Reihenfolge schichtweise befüllt:
1. 50 ml (21 cm) Edelstahlkugeln 1.4571, 3mm Durchmesser
2. 93,7 g Katalysator XT25367 von St. Gobain-NorPro (ergab eine Schütthöhe von 52cm)
3. Stahlsiebringe mit einem Durchmesser von ca. 5 mm und einer Höhe von ebenfalls ca. 5 mm bis zur Oberkante des Reaktors.

In der Schüttung waren Thermoelemente angebracht, die die Temperatur in der Katalysatorzone mißt. Der Reaktor wurde unter Stickstoff dergestalt elektrisch aufgeheizt, so daß die höchste Temperatur bei 350°C lag. Dann wurde von oben Isobutanol flüssig zudosiert, das in der ersten Zone verdampfte und gasförmig und auf ca. 350°C vorgeheizt auf die Katalysatorschüttung traf. Die zudosierten Mengen sind in den folgenden Tabellen angegeben. Über ein Druckhalteventil konnte der Druck, der sich durch die Umsetzung im Reaktor aufbaut, reguliert werden. Es wurden die Umsetzungen bei Normaldruck und bei 5 bara untersucht. Hierzu wurden aus der entspannten Produktgasatmosphäre über beheizte Leitungen Proben gezogen und diese hinsichtlich der gasförmigen Komponenten hin untersucht. Das Verhältnis Isobuten zu n-Butenen wurde durch eine Flächen-% Methode ermittelt, der Gesamtumsatz anhand der nicht umgesetzten organischen Bestandteile in der Gas- und der Flüssigphase ermittelt. Der Gesamtumsatz zu iso- und n-Butenen lag bei deutlich über 95%

**Tabelle 1**

| **Temp** | **Durchsatz** | **Druck** | **Iso-Buten** | **n-Butene** |
|---|---|---|---|---|
| **[°C]** | **[g/h]** | **[bara]** | **[area %]** | **[area %]** |
| 350 | 8,8 | 5 | 63,45 | 29,91 |
| 350 | 9,5 | 1 | 68,03 | 25,92 |
| 350 | 9,5 | 1 | 67,66 | 25,76 |
| 350 | 9,5 | 5 | 63,49 | 28,78 |
| 350 | 9,5 | 5 | 64.00 | 29,35 |

Tabelle 1 zeigt die Abhängigkeit des n-Buten zu Isobuten Verhältnisses als Funktion des Druckes bei Verwendung eines TiO₂-Katalysator XT 25376 von St. Gobain-NorPro unter Verwendung von 93,7g Katalysator (=Schütthöhe des Katalysatorbetts von 52 cm) in einem Aufbau wie in Fig. 1 beschrieben. Des weiteren ist in Tabelle 1 zu erkennen, daß die Druckabhängigkeit der n-Buten-Bildung unter diesen Bedingungen reversibel ist.

### Beispiel 2

In einem wie in Beispiel 1 beschriebenen Aufbau wurde anstelle des XT25376 vom Anatas-Typ ein TiO₂ vom Rutil-Typ (XT90045 von St-Gobain NorPro) mit einer BET-Oberfläche von <3 m²/g eingebaut. Es wurden für eine Katalysatorschüttung von 52 cm insgesamt 147,7g Katalysator eingesetzt.

Es wurden bei einem Durchsatz von 7,7 g/h unter einem Druck von 5 bara bei 350°C insgesamt 151,1g Isobutanol aufgegeben, die bei einem Umsatz von ca. 91% ein Leichtsiedergemisch mit einem Anteil von 77,7 % Isobuten und 21,5 % als Summe der n-Butene ergaben.

### Beispiel 3: Langzeitversuch mit XT25376

Eingesetzt wurde wie in Beispiel 1 ein Katalysator von St-Gobain-NorPro, Typ: XT 25376 aus TiO₂ mit einer BET-Oberfläche von ca. 140 m²/g und vom Anatas-Typ. Füllung des Reaktors wie in Beispiel 1. Der Reaktor wurde durchgehend bei einem Druck von 6,5 bara betrieben.

In das auf ca. 350°C resp. 375°C erhitzte Reaktorrohr (jeweilige Einstellung siehe Tabelle 2) wurde mit einer Rate von ca. 9 g/h kontinuierlich Isobutanol gepumpt. Nach einem Druckaufbau auf 6,5 bara wurde kontinuierlich Produkt abgenommen. Die Ergebnisse dieses Versuchs sind in Tabelle 2 dargestellt. Jeder der Versuchspunkte zeigt einen Umsatz von >98% bezogen auf das eingesetzte Isobutanol.

**Tabelle 2**

| **Tage nach Versuchsbeginn** | **Temp.** | **Durchsatz** | **iso-Buten** | **n-Butene** |
|---|---|---|---|---|
| | **[°C]** | **[g/h]** | **[area%]** | **[area%]** |
| 1 | 350 | 9,5 | 64,46 | 29,77 |
| 2 | 350 | 8,5 | 64,1 | 30,62 |
| 3 | 350 | 8,8 | 64,06 | 30,62 |
| 4 | 350 | 8,8 | 64,06 | 30,62 |
| 5 | 350 | 19,8 | 64,82 | 30,37 |
| 8 | 350 | 3,73 | 61,15 | 29,55 |
| 9 | 375 | 9,1 | 59 | 29,3 |
| 10 | 375 | 9,1 | 58,62 | 29,95 |
| 11 | 375 | 9 | 59,15 | 30,56 |
| 12 | 375 | 9,1 | 58,78 | 30,97 |
| 15 | 375 | 9,1 | 58,09 | 31,54 |
| 16 | 375 | 9 | 58,27 | 31,86 |
| 17 | 375 | 9 | 58,03 | 31,95 |
| 18 | 375 | 9,1 | 58,04 | 32,11 |
| 19 | 375 | 9,1 | 57,93 | 32,2 |
| 22 | 375 | 9,1 | 57,88 | 32,54 |
| 23 | 375 | 9,1 | 57,84 | 32,6 |
| 24 | 375 | 9,1 | 57,69 | 32,52 |
| 25 | 375 | 9 | 57,07 | 32,85 |
| 26 | 375 | 9,1 | 57,49 | 32,88 |
| 29 | 375 | 9,1 | 57,25 | 33,06 |
| 30 | 375 | 9 | 57,33 | 33,05 |
| 31 | 375 | 9,1 | 57,42 | 33,13 |
| 32 | 375 | 9 | 56,58 | 33,27 |
| 33 | 375 | 8,9 | 57,39 | 33,11 |
| 36 | 375 | 9,1 | 57,91 | 32,76 |
| 37 | 375 | 8,8 | 57,81 | 32,81 |

### Beispiel 4

In einem wie in Beispiel 1 beschriebenen Aufbau wurde anstelle des XT25376 ein Al₂O₃/SiO₂ - Mischoxid, das zu 24,3 Gew.-% aus Al₂O₃ und zu 74,2 Gew.-% aus SiO₂ besteht und eine BET-Oberfläche von 434 m²/g besaß (Hersteller: St-Gobain NorPro, Versuchsnummer 2006120085, DMS 2010 - 0380) eingesetzt. Es wurden für eine Katalysatorschüttung von 54 cm insgesamt 60,6g Katalysator eingesetzt.

In Tabelle 3 sind die Ergebnisse für diesen Katalysator aufgetragen. Die Angaben zum Umsatz beziehen sich hier auf die erhaltenen Reaktionswassermengen.

**Tabelle 3**

| **Temp.** | **Durchsatz** | **Durchsatz** | **Umsatz** | **iso-Buten** | **n-Butene** | **Wasser** |
|---|---|---|---|---|---|---|
| **[°C]** | **[g/h]** | **[g]** | **[%]** | **[area%]** | **[area%]** | **[g]** |
| 350 | 10,3 | 49,9 | ∼100 | 8,62 | 20,3 | 14,1 |
| 320 | 10,3 | 199,8 | 90,93 | 41,6 | 31,4 | 44,2 |
| 290 | 10,3 | 178,1 | 97,83 | 57,9 | 31,7 | 42,6 |
| 260 | 10,4 | 712,5 | 98,17 | 59,4 | 30,8 | 171 |
| 230 | 10,5 | 204,6 | 82,92 | 51,4 | 27,8 | 43,6 |

### Beispiel 5

In einem wie in Beispiel 1 beschriebenen Aufbau wurde anstelle des XT25376 ein TiO₂ Typ ST61120 von St-Gobain NorPro mit einer BET-Oberfläche von 156 m²/g eingebaut. Es wurden für eine Katalysatorschüttung von 51cm insgesamt 83,7g Katalysator eingesetzt.

In Tabelle 4 sind die Ergebnisse für diesen Katalysator aufgetragen. Die Angaben zum Umsatz beziehen sich hier auf die erhaltenen Reaktionswassermengen.

**Tabelle 4**

| Temp. | Durchsatz | Durchsatz | Umsatz | iso-Buten | n-Butene | Wasser |
|---|---|---|---|---|---|---|
| **[°C]** | **[g/h]** | **[g]** | **[%]** | **[area%]** | **[area%]** | **[g]** |
| 350 | 8,6 | 41,6 | ∼100 | 55,4 | 26,5 | 11,1 |
| 320 | 8,6 | 165,3 | 84,54 | 63,9 | 29,9 | 34 |
| 290 | 8,6 | 148 | 43,51 | 69,7 | 25,7 | 16,8 |

## Patentansprüche

1. Verfahren zur Dehydratisierung von Isobutanol zu linearen n-Butenen, **dadurch gekennzeichnet, dass** als Katalysator in einem Festbett TiO₂ mit einer BET-Oberfläche > 10m²/g oder ein Al₂O₃/SiO₂ eingesetzt werden.

2. Verfahren gemäß Anspruch 1, **dadurch gekennzeichnet, dass** als Katalysator TiO₂ mit einer BET-Oberfläche> 10m²/g, bevorzugt 20 bis 400 m²/g, eingesetzt wird.

3. Verfahren gemäß Anspruch 1, **dadurch gekennzeichnet, dass** als Katalysator Al₂O₃/SiO₂ mit einer BET-Oberfläche 0,1 bis 100m²/g eingesetzt wird.

4. Verfahren gemäß Anspruch 3, **dadurch gekennzeichnet, dass** als Katalysator Al₂O₃/SiO₂ mit einem Gehalt an SiO₂ im Bereich von 0,1 bis 80 Gew.-% eingesetzt wird.

5. Verfahren gemäß Anspruch 2, **dadurch gekennzeichnet, dass** als Katalysator TiO₂ vom Typ Anatas eingesetzt wird.

6. Verfahren gemäß der Ansprüche 1 bis 5, **dadurch gekennzeichnet, dass** das Festbett Teil eines Rohrbündelreaktors oder Röhrenreaktors ist.

7. Verfahren gemäß Anspruch 6, **dadurch gekennzeichnet, dass** der Reaktor aus Stahl oder Edelstahl ist.

8. Verfahren gemäß einem der Ansprüche 1 bis 7, **dadurch gekennzeichnet, dass** dieses bei Temperaturen im Bereich von etwa 200 bis 500°C, vorzugsweise 250 bis 400°C durchgeführt wird.

9. Verfahren gemäß der Ansprüche 1 bis 8, **dadurch gekennzeichnet, dass** der Reaktor bei einem Druck von 1 bis 300 bar betrieben wird.

10. Verfahren gemäß der Ansprüche 1 bis 9, **dadurch gekennzeichnet, dass** als Edukt Isobutanol aus mikrobiellen und/oder fermentativen Prozessen eingesetzt wird.

11. Verfahren gemäß der Ansprüche 1 bis 10, **dadurch gekennzeichnet, dass** als n-Buten Buten-1 gewonnen wird.

12. Verfahren gemäß einem der Ansprüche 1 bis 11, **dadurch gekennzeichnet, dass** dieses kontinuierlich betrieben wird.
